# EUROPEAN PATENT APPLICATION

(11) **EP 4 847 011 A1**
(43) Date of publication of application: **02.12.2026**
(21) Application number: 25179812.0
(22) Date of filing: 30.05.2025
(51) Int. Cl.: A61K 9/00, A61K 47/36, A61K 47/44

(54) **OPHTHALMIC COMPOSITIONS COMPRISING ASTAXANTHIN IN COMBINATION WITH HYALURONIC ACID AND ETHOXYLATED HYDROGENATED CASTOR OIL**

(71) Applicant: Delim Cosmetics & Pharma S.r.l., 20124 Milano (IT); Aveflor Axt s.r.o., 507 32 Budceves (CZ)
(72) Inventor: RONCHI, FEDERICA, 20124 MILANO (IT); RONCHI, CELESTINO, 20124 MILANO (IT)
(74) Representative: Bianchetti & Minoja with Trevisan & Cuonzo IPS SRL

(57) **Abstract**

The present invention relates to ophthalmic compositions comprising astaxanthin or a phytocomplex thereof in association with hyaluronic acid and ethoxylated hydrogenated castor oil. The compositions of the invention are useful for the treatment of dry eye.

## Description

The present invention relates to ophthalmic compositions comprising astaxanthin or a phytocomplex thereof in association with hyaluronic acid and ethoxylated hydrogenated castor oil. The compositions of the invention are useful for the treatment of dry eye.

### Background of the invention

Astaxanthin, a naturally occurring carotenoid pigment, is commonly found in various plants, microalgae, fishes and crustaceans (e.g. salmon, shrimp, lobster, crab) *(*Ambati RR, Phang SM, Ravi S, Aswathanarayana RG. Astaxanthin: sources, extraction, stability, biological activities and its commercial applications--a review. Mar Drugs. 2014 Jan 7;12(1):128-52*).*

Astaxanthin is often found in association with other carotenoids, such as beta-carotene, lutein and zeaxanthin, as well as with other natural antioxidants such as vitamin E, vitamin C and polyphenols. Astaxanthin is known for its powerful antioxidant properties, having 100- fold higher antioxidant activity than α- tocopherol *(*Giannaccare G. et al., Clinical Applications of Astaxanthin in the Treatment of Ocular Diseases: Emerging Insights- a review. Mar Drugs. 2020, May 18, 239*).*

CZ2019799A3, in the name of the co-applicant Aveflor, discloses the preparation of an astaxanthin-based phytocomplex from microalgae biomass. The phytocomplex is obtained in high yield and consists of a mixture of free and esterified astaxanthin, substantially free from by-products of microalgae metabolism. The process involves the controlled cultivation of astaxanthin-rich algae, such as *Haematococcus pluvialis,* under optimal conditions to maximize pigment production. Once the algal biomass has been harvested, it undergoes a drying process to reduce moisture content, facilitating subsequent extraction steps.

Next, the dried biomass is treated with selected organic solvents, such as ethanol or acetone, under specific temperature and pressure conditions to solubilize the astaxanthin contained in the algal cells. To enhance extraction efficiency, the method may include pre-treatment techniques, such as high-pressure homogenization or ultrasound application, which help the fracture of the algal cell walls, increasing the availability of astaxanthin for extraction.

After extraction, the astaxanthin-solvent mixture undergoes separation processes, such as filtration or centrifugation, to remove solid residues. The solvent is then evaporated, yielding a concentrated astaxanthin extract.

CZ2019799 also proposes methods for purifying the extracted astaxanthin, using techniques such as chromatography to obtain a high-purity product, providing an efficient and sustainable approach for producing high-quality astaxanthin from natural sources.

### Description of the invention

It has now been found that the association of astaxanthin or a phytocomplex thereof with hyaluronic acid and ethoxylated hydrogenated castor oil allows the preparation of stable ophthalmic compositions having antioxidant and lubricating power useful for improving dry eye and other ophthalmic disorders.

The invention accordingly provides ophthalmic compositions comprising astaxanthin in combination with hyaluronic acid or a salt thereof and ethoxylated hydrogenated castor oil. The compositions of the invention are preferably in form of aqueous nano-suspensions.

The concentration of astaxanthin, preferably all-trans astaxanthin, ranges from 0.001 to 1 % by weight, preferably from 0.01 to 0.5% by weight.

Any form of astaxanthin may be used according to the invention, even though it is preferred the phytocomplex prepared according to CZ 2019799. Said phytocomplex contain different phytochemicals and ingredients acting synergistically.

Hyaluronic acid or a salt thereof, preferably sodium hyaluronate, may be linear or cross-linked, having average molecular weight, determined by HPLC, from 200 to 2000 kDa, preferably from 250 to 1200 kDa. Suitable cross-linked hyaluronic acid may be obtained by reaction with cross-linking agents such as methacrylamide, hydrazide, carbodiimide, divinyl sulfone, 1,4-butanediol diglycidyl ether (BDDE), poly(ethylene glycol) diglycidyl ether, panthenol or propylbisoxyamine.

Hyaluronic acid or a salt thereof is present at a concentration between 0,01 and 10% by weight, preferably between 0,1 and 1% by weight.

Ethoxylated hydrogenated castor oil, marketed by BASF as Kolliphor^{®} RH40, is present in the compositions of the invention in the range from 3 %- 5% w/w.

The compositions of the invention, either for human or veterinary use, may comprise pH stabilizers, antioxidants, humectants, chelating agents, permeation enhancers and are preferably free from preservatives.

To ensure good compliance, the compositions of the invention must be isotonic (i.e. same osmolarity of a 0.9% w/w physiological solution), with low viscosity and pH of about 7,0 ± 0,5.

Suitable administration forms include eye drops or any other form suitable for topical application into the eye, in particular for the treatment of dry eye.
as an ophthalmic nano-suspension in an aqueous vehicle.

The following example illustrates the invention in more detail.

### Example: ophthalmic nano-suspension (eye drops)

### Composition

**Table 1: quali-quantitative formulation**

| *Compound:* | *For 100g:* |
|---|---|
| Astaxanthin | 0,01 |
| Kolliphor^{®} RH40 | 3,00 |
| Butylated hydroxytoluene | 0,10 |
| Hydrogen phosphate bipotassium | 0,23 |
| Dihydrogen phosphate potassium | 0,18 |
| Glycerol | 1,65 |
| Sodium edetate | 0,20 |
| Hyaluronic acid | 0,40 |
| Water for injection | Up to 100 |

### Manufacturing method

The manufacturing process comprises:
- Preparation of oil phase: Kolliphor^{®} RH40, BHT, astaxanthin;
- Preparation of water phase: water for injection, phosphate buffer, sodium edetate, hyaluronic acid, glycerol;
- Dispersion of astaxanthin suspension in the aqueous phase;
- Insufflation of nitrogen in the final packaging.

The pH, osmolarity, Zeta potential and assay of the nano-suspension have been measured The pH was tested using the liquid probe of the pH-meter Cyberscan pH 110, Eutech Instruments. The sample was kept in a climatic chamber stabilized at temperature of 25 ± 2 °C. The reading was carried out when a stable value was obtained. The value registered was about 6,8, compatible with eyes environment.

The osmolarity was tested using an osmometer Fiske^{®} Micro-Osmometer Model 21. The sample was kept in a climatic chamber stabilized at a temperature of 25 ± 2 °C. The reading was carried out when a stable value was obtained and it was compared with the value registered for a physiological solution (i.e. NaCl 0,9 % w/w). The value recorded for the physiological solution was 287 mOSM/kg. The value registered for the eye drops was 280 mOSM/Kg, i.e. substantially isotonic.

### Measurement of Zeta potential:

Zeta potential is a measure of the surface electric charge of particles suspended in a liquid and is used to assess the stability of colloidal dispersions and emulsions. It represents the difference in electric potential between the slipping plane and the surrounding fluid in the mobile layer. The higher its absolute value, the more the particles tend to repel each other, thus increasing the stability of the suspension. The measurement is based on laser Doppler electrophoresis or micro-electrophoretic electrophoresis, according to the following steps:
- The solution containing the particles is diluted, if necessary, to ensure accurate measurement.
- The suspension is placed in a cell with two electrodes. An electric field is applied, inducing the movement of charged particles.
- A laser illuminates the suspension, and the scattered light is analyzed using the Doppler effect technique.
- The movement of charged particles in the electric field causes a shift in the frequency of the reflected light.
- The migration velocity of the particles is converted into electrophoretic mobility.
- Using Smoluchowski's equation (or other formulas depending on the system), the zeta potential is calculated in millivolts (mV).

The Zeta potential is influenced by the following factors:
- pH: it modifies the surface charge of the particles.
- Ionic strength: the salts present in the solution shield the charges.
- Additives (polymers, surfactants): they can alter the surface of the particles.

The Zeta Sizer also provides information on electrophoretic mobility, expressed in µm cm/Vs (micrometers per centimeter per volt-second). This measurement represents the speed at which particles move in the applied electric field and is closely related to the zeta potential.

A study was conducted to analyze the impact of different amounts of surfactant (i.e. Kolliphor^{®} RH40), while keeping the formulation constant, on the stability of suspensions. Five suspensions with increasing amounts of surfactant were prepared, added to the cell and analyzed.

The preparations are as follows:
- Preparation A: 0,5% w/w of Kolliphor^{®} RH40, 0,01% w/w of astaxanthin, water for injection up to 100% w/w
- Preparation B: 1,0% w/w of Kolliphor^{®} RH40, 0,01% w/w of astaxanthin, water for injection up to 100% w/w
- Preparation C: 2,0% w/w of Kolliphor^{®} RH40, 0,01% w/w of astaxanthin, water for injection up to 100% w/w
- Preparation D: 3,0% w/w of Kolliphor^{®} RH40, 0,01% w/w of astaxanthin, water for injection up to 100% w/w
- Preparation E: 5,0% w/w of s Kolliphor^{®} RH40, 0,01% w/w of astaxanthin, water for injection up to 100% w/w.

### Analysis parameters:

- Instrument: Zetasizer Pro from Malvern-Panalytical
- Technique: Electrophoretic Light Scattering (ELS)
- Measurement cell: DTS1070
- Detector: Forward scattering at 13°
- Voltage: Automatic
- Attenuator: Automatic
- Analysis model: Automatic
- Pause between measurements: 60s
- Dispersant: Water
- Dispersant viscosity and refractive index: 0,8872 cp and 1.330 (water)
- Dispersant dielectric constant: 78.5
- Temperature: 25°
- Temperature equilibration time: 120 s
- Number of measurements: 3.

The following table shows the mean results of Z potential and electrophoretic mobility obtained from the average of three measurements for each sample:

**Table 2: Mean results of Z potential and electrophoretic mobility for each preparation (A,B,C,D)**

| Sample | Z-potential (mV) | Mobility (µmcm/Vs) |
|---|---|---|
| Preparation A | -10,22 | -0,80 |
| Preparation B | -7,75 | -0,61 |
| Preparation C | -12,00 | -0,94 |
| Preparation D | -25,88 | -2,03 |
| Preparation E | -27,59 | -2,16 |

The Reference values for Z potential and Electrophoretic Mobility for data interpretation are shown in the following table:

**Table 3: Z potential reference values and their interpretation**

| Z-potential (mV) Reference values | Data interpretation: |
|---|---|
| 0 - ± 10 | Very unstable, high tendency to aggregate |
| ±10 - ±30 | Moderate stability |
| ±30 - ±60 | Good stability |
| >±60 | High stability, strong particle repulsion |

**Table 4: Electrophoretic mobility reference values and their interpretation**

| Electrophoretic mobility (µmcm/Vs) Reference values | Data interpretation: |
|---|---|
| **0 - ±0.4** | Very unstable, high tendency to aggregate |
| **±0.4** - **±1.0** | Moderate stability, possible slow aggregation or coalescence |
| **±1.0** - **±2.0** | Good stability, effective electrostatic repulsion |
| > **±2.0** | High stability, strong particle repulsion |

Suspensions with a Kollidon^{®} RH40 concentration equal or greater than 3% w/w are stable with a low tendency for particle aggregation.

### Assay:

The analysis has been performed under the following conditions.

### Chromatographic conditions

- Instrument: Agilent 1100 HPLC System, with Diode Array Detector G1315B, Agilent.
- Software: OpenLab ChemStation 2.3, Agilent
- Column: Phenomenex, Kinetex^{®} C18 EVO 10cm x 4,6mm, 5µm ,100Å
- Column temperature: 40 °C
- Flow: 1,5mL/min
- Injection volume: 10µL
- Autosampler temperature: 19 °C
- Run time: 15min
- Detection wavelengths: 480nm
- Mobile Phase A: 80% methanol 20% water
- Mobile Phase B: methanol
- Gradient:

**Table 5: Gradient ofHPLC method**

| Time (min) | Mobile phase A (% V/V) | Mobile phase B (% V/V) |
|---|---|---|
| 0 | 100 | 0 |
| 2 | 100 | 0 |
| 7 | 0 | 100 |
| 12 | 0 | 100 |
| 13 | 100 | 0 |
| 15 | 100 | 0 |

### Preparation

***All-trans* astaxanthin standard solution (AXT STD=** 200µg/ml AXT): 10±0,1mg of All-*trans* astaxanthin standard were dissolved in 20mL of acetone in a 50mL volumetric flask and diluted to volume with acetone.

The calibration curve is one of the methods for evaluating the concentration of an analyte in an unknown sample. The goodness of fit was evaluated through linear regression coefficient R². **Eye drops test solution (ED-TS):** 2 ± 0,1g of eye drops were added in a 5ml volumetric flask and diluted to volume with diluent. After stirring for 5min and sonication for 5min, hyaluronic acid precipitated. An aliquot of supernatant was sampled and filtered through 0.45µm Nylon filter prior to analysis.

**Table 6: Recovery of Astaxanthin in ED-TS**

| | Weight (mg) | Area (mAU*s) | Conc (µg/mL) AXT | % w/w AXT |
|---|---|---|---|---|
| I | 2,0509 | 2143,7 | 44,36 | 0,0108 |
| II | 2,0141 | 2038,8 | 42,00 | 0,0104 |
| III | 2,0252 | 2012,8 | 41,42 | 0,0102 |
| IV | 2,0172 | 2036,4 | 41,95 | 0,0104 |
| VI | 2,0066 | 2038,4 | 41,99 | 0,0105 |
| VI | 2,0178 | 2050,9 | 42,28 | 0,0105 |
| | **Mean** | 2053,5 | 42,33 | **0,010*** |
| | **Std.Dev** | 41,90 | 0,94 | **0,0002** |
| | **%RSD** | 2,04 | 2,22 | **1,68** |

| | | | | |
|---|---|---|---|---|
| **Corresponding to 100% of recovery* | | | | |

The content in free *all-trans* astaxanthin of eye drops (in finished dosage form) was 0,0105±0,0002% and within the acceptance criteria.

An RSD% ≤ 5% of the area of the phytocomplex peaks indicates good precision of the analytical procedure.

## Claims

1. Ophthalmic compositions comprising astaxanthin in combination with hyaluronic acid or a salt thereof and ethoxylated hydrogenated castor oil.

2. Compositions according to claim 1 in form of nano-suspensions.

3. Compositions according to claim 1 or 2 wherein hyaluronic acid is linear or cross-linked having average molecular weight, as determined by HPLC, from 200 to 2000 kDa, preferably from 250 to 1200 kDa.

4. Compositions according to any one of claims 1-3, wherein hyaluronic acid is present at a concentration between 0,01 and 10% by weight, preferably between 0,1 and 1% by weight.

5. Composition according to any one of claims 1-4 free from preservatives.

6. Compositions according to any one of claims 1-5 comprising pH stabilizers, antioxidants, humectants, chelating agents, permeation enhancers.

7. Composition of claims 1-6 for use in the treatment of dry eye.
